(19) Europäisches Patentamt
European Patent Office
Office européen des brevets

(11) **EP 4 737 559 A1**

(12) **EUROPEAN PATENT APPLICATION**

(43) Date of publication:
06.05.2026  Bulletin 2026/19

(21) Application number: 25209608.6

(22) Date of filing: 17.10.2025

(51) International Patent Classification (IPC):
*C12N 5/071* (2010.01)   *G01N 15/08* (2006.01)
*G01N 33/50* (2006.01)   *C07K 14/78* (2006.01)
*C12M 1/12* (2006.01)

(52) Cooperative Patent Classification (CPC):
**C12N 5/0698; C07K 14/4741; C12M 25/14;
G01N 33/5088;** C12N 2533/40; C12N 2533/50;
G01N 15/0806

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 17.10.2024  PT 2024119779
10.03.2025  PT 2025120101

(72) Inventors:
• BARBOSA FERNANDES, EDUARDA
4710-057 BRAGA (PT)
• DIONÍSIO LÚCIO, MARLENE SUSANA
4710-057 BRAGA (PT)
• FERNANDES CARDOSO, VANESSA
4800-058 GUIMARÃES (PT)
• LANCEROS-MENDEZ, SENENTXU
4710-057 BRAGA (PT)
• MARTINS LOPES, CARLA
4249-004 PORTO (PT)

(71) Applicants:
• Universidade do Minho
4704-553 Braga (PT)
• Universidade Do Porto
4099-002 Porto (PT)
• Fundação Ensino e Cultura Fernando Pessoa
4249-004 Porto (PT)

(74) Representative: Patentree
Edificio Net
Rua de Salazares, 842
4149-002 Porto (PT)

(54) **SKIN MODEL FOR SYNCHRONIZING STUDIES OF PERMEATION AND MEMBRANE INTERACTIONS**

(57)    The present disclosure relates to an artificial skin model that mimics the intercellular lipid matrix of the *stratum corneum* in healthy or injured skin, including the short and long lamellar periodicity phases that are thought to be essential to the barrier function of the skin. In particular, the present disclosure relates to simple and cost-effective methods and processes for high through-put screening (HTS) of skin permeation assays and membrane interaction studies, particularly useful in the pharmaceutical, cosmetic, and chemical industries for the evaluation of topical formulations, transdermal delivery systems, and other skin-related treatments.

EP 4 737 559 A1

## Description

### TECHNICAL FIELD

The present disclosure relates to an artificial skin model comprising a polymeric scaffold that supports a mixture that mimics the stratum corneum intercellular lipid matrix of healthy or injured skin. More specifically, the present disclosure relates to an artificial bioinspired skin model for skin permeation assay and for the study of molecular interactions and biophysical changes in the *stratum corneum* intercellular lipid matrix.

### BACKGROUND

Skin barrier properties are vital in various research fields, including toxicology, risk assessment, and pharmaceutical and cosmetic product development. Delivering drugs or bioactive ingredients through the skin offers numerous advantages over traditional routes like oral or injectable [1]. However, the natural barrier function of the skin mainly due to the stratum corneum complex organization presents challenges to permeation.

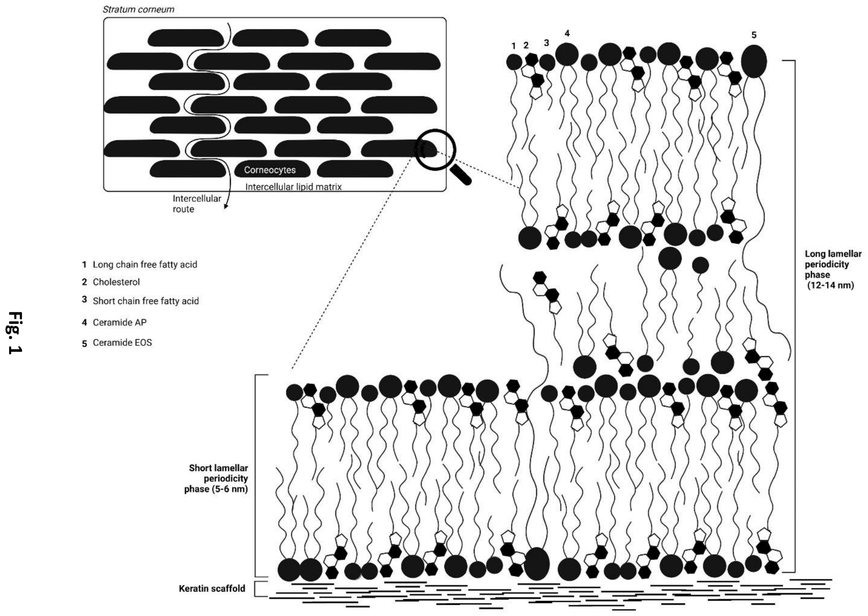

The stratum corneum consists of corneocytes embedded by an intercellular lipid matrix, in a 'brick-and-mortar' wall like structure (Fig. 1) [2, 3]. Lamellar and lateral organization is crucial for skin barrier function and integrity of the intercellular lipid matrix. Healthy intercellular lipid matrix of the stratum corneum is organized in a dense orthorhombic lateral packing with two lamellar phases (long and short lamellar periodicity phases) with repeat distances of 12-14 and 5-6 nm respectively (Fig. 1) [2, 4]. Injured skin (like atopic dermatitis skin) has impaired skin barrier due to changes in the intercellular lipid matrix from orthorhombic to a loosely hexagonal lateral packing [4].

Using skin models able to replicate the composition, structure and barrier properties of the intercellular lipid matrix is essential for assessing the percutaneous absorption, bioavailability, and safety of topically applied cosmetic ingredients and dermatological bioactive ingredients.

The use of skin, whether human or animal, has long been regarded as the gold standard for assessing (trans) dermal permeability. Commercially available products like Transderm-Scop®, Androderm®, and Alora® are excised human skin used in permeation assays as ex vivo skin models. Yet, ethical, technical, and economic challenges have stalled its widespread use. Obtaining human skin poses hurdles due to ethical concerns, high variability, low throughput, and technical challenges, limiting its use [5, 6]. Similarly, obtaining animal skin is time-consuming, technically challenging, and costly, and excised skins do not accurately represent human skin physiology and structure [6]. Besides, both in vivo animal and human studies and ex vivo studies using excised skin models contribute significantly to the carbon footprint of pharmaceutical industries raising sustainability concerns. The pharmaceutical and industry/research sectors are increasingly inclined to adopt other *in vitro* testing methods to lessen reliance on *in vivo* or *ex vivo* models. This trend is further emphasized by the EU prohibition on animal testing for cosmetics and toxicology evaluations under Directive 76/768/EEC, which demands for alternative methods of skin permeation testing [7].

Models equivalent to the living skin developed by culturing human skin components such as keratinocytes and fibroblasts, and several human skin cell cultures are commercially available as skin models: EpiSkin™ model (Episkin, company of group L'Oréal, Lyon, France); EpiDerm™ model (MatTek Corporation, Ashland,MA, USA); SkinEthic™ (Episkin, company of group L'Oréal, Lyon, France); Labskin™ (Labskin, Lyon, France); EpiCS® model (CellSystems, Troisdorf, Germany); Straticell model (Straticell, Les Isnes, Belgium); and Labcyte model (Gamagori, Japan); Full-Thickness models such as a StrataTest® model (Stratatech, Madison, WI, USA); Phenion Full-Thickness Skin Model (Phenion, Düsseldorf, Germany); GraftSkin® (Apligraf; Organogenesis, MI, USA); EpiDermFT® (MatTek Corporation, Ashland, MA, USA); and Vitrolife-Skin™ model (Kyoto, Japan). These models have been employed in Franz diffusion cells for permeation assessments or incorporated into microfluidic chips (known as "skin-on-chip"). However, they still fail to address several issues related to excised tissue, proving expensive, requiring specialized technical skills, and inadequately replicating human skin permeation, and results with these models frequently show large variations [8, 9]. Primarily utilized for skin irritation and toxicity assessments [10], these cellular models present challenges that surpass the conveniences offered by microfluidic analysis and screening purposes. Despite the potential benefits of microfluidic technology in enhancing screening processes, the integration of skin cell-based models presents obstacles to achieving high throughput screening (HTS). These include limitations in storage capacity of the cell-based models, lack of reproducibility, technical handling requirements, and elevated screening costs.

Cell-free skin models have also been developed to access skin permeation. Most models are synthetic membranes for transdermal delivery composed of silicone-based membranes such as Silatos™ (LMA, Better by design, London, UK), Silastic® (Dow Corning Corporation, Michigan, USA), and multiple layers of polyester sulfone Strat-M® (Merck Millipore, Burlington, Massachusetts, USA). These models, predominantly composed of hydrophobic polymers, exhibit varying permeation efficiencies. Establishing a reliable correlation with the barrier function of the human stratum corneum, especially in finite dose applications, remains a challenge. While they provide estimations for hydrophilic compounds, they overestimate the permeation of lipophilic compounds [6]. Addressing this discrepancy is essential for improving the precision of permeation estimations.

Other cell-free based models such as Permeapad® barrier (PHABIOC GmbH, Karlsruhe, Germany holding international patent WO2016078667) features two cellulose membranes with a phospholipid layer in between, while Skin-PAMPA™ (Pion Inc, Billerica, USA) utilizes a model containing a synthetic amide-based compound, free fatty acid, and cholesterol in the Parallel Artificial Membrane Permeability Assay (PAMPA). Permeapad® barrier offers a membrane model for use in Franz diffusion cells or as part of the PermeaPad® Plate, consisting of two multiwell plates. Skin-PAMPA™ is available as the SuperQuick® Skin-PAMPA Kit (Creative bioarray, New York, USA) also comprising two multiwell plates. The upper plate serves as the donor, with an insert plate integrating the membrane model (in the case of PermeaPad® Plate) or the skin model is provided as an organic solution to be added to the donor plate and allowed to dry (in the case of Skin-PAMPA™). The bottom plate acts as the acceptor and can be placed in standard fixtures of multiwell plates from High-performance liquid chromatography (HPLC) equipment to quantify permeated compounds.

Cell-free PermeaPad® and Skin-PAMPA™ offer advantages over cell-based models, including better reproducibility and ease of handling. However, they still lack important elements to mimic *stratum corneum,* such as the incorporation of skin proteins or consideration of the skin protein-lipid ratio. Additionally, they fail to replicate the long and short lamellar periodicity phases crucial for the lamellar and lateral organization of the stratum corneum, essential for skin permeation barrier functions [2,4]. Furthermore, Skin-PAMPA™ lacks the ability to be removed, after permeation assays, to study the molecular interactions and biophysical changes that occurred in the stratum corneum intercellular lipid matrix. Although PermeaPad® barrier can be dissolved post-permeation to extract bioactive ingredients with ethanol or methanol by splitting the cellulose scaffolds, this process destroys the lipid assembly, rendering post-permeation biophysical investigations impossible. Neither the Skin-PAMPA™ nor the PermeaPad® barrier models can establish correlations between molecular structures and permeation efficacy, as well as evaluate the potential skin toxicity.

There is also a gap in evaluating the percutaneous permeation in injured skin, crucial for tailoring treatments to specific patient needs. None of the cell-free skin models replicate injured skin conditions.

The present disclosure relates to a bioinspired skin model that solves the above-mentioned limitations and differentiates from all other models: (i) it mimics the two-periodicity structure of intercellular lipid matrix of the stratum corneum; (ii) the lipid matrix can be assembled on a keratin scaffold that creates a more representative skin protein:lipid ratio; (iii) provides models for healthy and injured skin (such as atopic dermatitis), addressing a gap in current research and enabling evaluation of permeation in injured skins, aligning with personalized medicine; (iv) skin cell-free models can be removed after permeation assays and the molecular interactions and biophysical changes in stratum corneum intercellular lipid matrix can be analysed, adding a new dimension to skin model evaluation.

These facts are disclosed in order to illustrate the technical problem addressed by the present disclosure.

## GENERAL DESCRIPTION

The present disclosure relates to an artificial skin model that mimics the intercellular lipid matrix of the stratum corneum in healthy or injured skin, including the short and long lamellar periodicity phases that are thought to be essential to the barrier function of the skin.

The present disclosure relates to methods and systems for performing high-throughput screening (HTS) of skin permeation assays. More specifically, the disclosure introduces models that account for diverse skin conditions, such as atopic dermatitis, to facilitate personalized treatment development.

In an aspect of the present disclosure, the bioinspired mimetic model may comprise a lipid bilayer supported on a polymeric scaffold, which allows it to be portable and removable to evaluate the molecular interactions and biophysical changes induced after permeability studies. The present disclosure is designed to be used in skin permeability and membrane interaction studies, and is compatible with traditional Franz diffusion cells and other permeability assay constructs, as well as high throughput screening (HTS) assays like microfluidic constructs.

### Beneficial aspects of the present disclosure

An aspect of the present disclosure introduces innovative features that push the boundaries of skin permeation research. The present disclosure is cell-free providing increased reproducibility and ease of handling. In an era where sustainability, efficiency, and ethical issues are essential, the focus should be on replacing human, animal tissues, and the present disclosure aligns perfectly with these global priorities. Moreover, with the EU ban on the use of animals in cosmetic testing there is an increased demand for developing and validating alternative skin permeation methods before clinical tests.

Surprisingly, one key benefit of the present disclosure is the assembly of intercellular lipid matrix of the stratum corneum (mimicking the short and long lamellar periodicity phases that are thought to be essential to the barrier function of the skin) on a polymeric scaffold for creating a more representative skin protein-to-lipid ratio, enhancing the model's physiological relevance (Fig. 1).

Another beneficial aspect of the present disclosure lies in its capacity to address the diverse spectrum of skin conditions encountered in real-world scenarios. While traditional models primarily focus on evaluating compound permeation in healthy skin, the present disclosure introduces a paradigm shift by incorporating injured skin models representing conditions such as atopic dermatitis. This inclusion allows researchers to explore how various skin conditions impact the permeation of compounds and formulations, offering insights into the efficacy and safety of treatments tailored to individual patient profiles. By simulating the permeation behaviour in injured skin, the present disclosure aligns with the principles of personalized medicine, which emphasizes customized treatments based on individual patient characteristics and needs. This innovative approach enables researchers to better understand how specific skin conditions affect the barrier function and permeability of the skin, thereby facilitating the development of targeted therapies. Beyond atopic dermatitis, the present disclosure provides a foundation for the development of models representing other skin conditions. This versatility ensures that researchers can explore a wide range of dermatological disorders, opening doors for further advancements in personalized medicine and skin care research.

Another beneficial aspect of the present disclosure is the fact that the bioinspired skin model can be removed enabling post-permeation analysis of molecular interactions and biophysical changes occurring at the skin model. This feature provides researchers with a deeper insight into the mechanisms underlying permeation. By examining how different compounds interact with the bioinspired skin model at a molecular level, scientists can better understand the factors influencing permeation rates and efficiency. This deeper understanding is crucial for optimizing the development of (trans)dermal bioactive ingredients and formulations, as it allows for the identification of key molecular characteristics that contribute to enhanced permeation. Moreover, the ability to conduct post-permeation analysis also facilitates the identification of any adverse effects or skin toxicity resulting from compound permeation. If the analysis reveals that certain compounds have disrupted the biophysical integrity of the model, indicating potential toxicity or adverse reactions, researchers can take appropriate measures to modify or eliminate these compounds from further consideration. This proactive approach to assessing skin toxicity is essential for ensuring the safety and efficacy of (trans)dermal products, ultimately leading to improved consumer health outcomes.

## Components of the disclosure and assembly methods

The present disclosure is a bioinspired skin model comprising a polymeric scaffold that supports a lipid bilayer in an assembly that mimics the protein:lipid ratio found in the skin. These components and their assembly are described in the next sections.

## Polymeric scaffold

In an embodiment, the polymeric scaffold is highly permeable, having a porosity ratio from 20% to 95% with a pore size inferior to 650 nm and allowing the phospholipid layer to have the primary influence on permeability. The porosity was measured, based on a method previously reported [11]. Briefly, scanning electron microscopy (SEM) images were used to measure the thickness of the polymeric scaffold, which enables to estimate the fibrous matrix porosity (p), using equation (1):

$$p = 100(1 - (\rho fibers / \rho PCL)) \hspace{4cm} Eq.\ (1)$$

where $\rho_{PCL}$ is the density of PCL (1.145 g/cm$^3$ as indicated by the supplier) and $\rho_{fibers}$ is the effective density of the fiber mats, obtained by dividing the mass of a sample by its volume.

In an embodiment, pore size of the polymeric scaffold was determined from scanning electron microscopy (SEM) images using image analysis software (e.g., ImageJ®). Briefly, high-resolution SEM micrographs were acquired at different magnifications and representative areas. The pore diameter was determined by fitting each pore area to a circle of equal area, and the pore size was obtained from the average of at least 50 measured pores across different regions of the sample.

Sheets made of a hydrophilic mixed cellulose esters or polycarbonate have been provided as an illustrative example in the given section.

In an embodiment, the polymeric scaffold may comprise at least one a sheet of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof. The polymeric scaffold is not restricted to any material.

In another embodiment, the polymeric scaffold may comprise at least one layer of keratin over the surface of a sheet of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof.

In another embodiment, the polymeric scaffold may comprise electrospun fibers of polyvinyl alcohol, polylactic-co-glycolic acid, polylactic acid, polycaprolactone, polyethylene oxide, polyvinylpyrrolidone, poly(methyl methacrylate), ethyl cellulose, or composites thereof.

In another embodiment, the polymeric scaffold may comprise electrospun fibers of keratin and keratin derivatives from different sources (such as extracted from woven and non-woven sources).

In an embodiment, keratin derivatives are obtained from various sources, including woven keratinous materials (e.g., wool fabrics) and non-woven keratinous materials (e.g., hair, feathers, or nails).

In another embodiment, the polymeric scaffold may comprise electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose.

In another embodiment, the polymeric scaffold may comprise two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose.

In another embodiment, the polymeric scaffold may comprise three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose.

In an embodiment, the thickness of the polymeric scaffold ranges from 10 $\mu$m - 400 $\mu$m, preferably 10 $\mu$m - 60 $\mu$m.

**Supported lipid bilayers**

In an embodiment, the supported lipid bilayer may be composed of different lipid classes, cholesterol and free fatty acids resembling the composition of the human intercellular lipid matrix stratum corneum. The lipid composition and ratios can be diverse, as long as they are organized in lipid bilayers with lateral packing and lamellar periodicity phases that mimic healthy or injured skin (like atopic dermatitis skin).

In an embodiment, the supported lipid bilayer may comprise cholesterol (from 0% up to 35%) and phosphatidylcholine or its diacyl derivatives (from 65% up to 100%): from C(12) to C(24) fatty acyl substituted glycero-3-phosphocholines. Supported lipid bilayers composed of cholesterol (0% or 33%) and C(16) fatty acyl derivative: 1,2-dipalmitoyl-sn-glycero-3-phosphocholine (100% or 67%) have been provided as an illustrative example in the given section.

In another embodiment, the healthy skin model contains a supported lipid bilayer composed by acylceramides (ceramide AP and ceramide EOS), cholesterol and free fatty acids (23:10:33:33). Free fatty acids will be a mixture of arachidonic acid:behenic acid:lignoceric acid: hexacosanoic acid:myristic acid in molar ratios of (3:16:12:1:1) $\pm$ (0.3:1.7:1.3:0.1:0.5).

As used herein, Ceramide AP is a type of sphingolipid corresponding to N-($\alpha$-hydroxyacyl)-phytosphingosine, typically found in the stratum corneum of the skin. Ceramide AP may be of natural origin (e.g., extracted from animal or plant sources) or synthetically produced through chemical or biotechnological processes. The ceramide AP may be included as such or in the form of a derivative, analog, or salt thereof Ceramide AP is a naturally occurring component of the stratum corneum lipids and contributes to epidermal barrier function and skin hydration by reinforcing the lamellar lipid structure.

As used herein, Ceramide EOS corresponds to N-(acyl)-$\omega$-hydroxyacyl-sphingosine, a subclass of ceramides containing a long-chain $\omega$-hydroxy fatty acid esterified with linoleic acid. The ceramide EOS may be obtained from natural or synthetic sources, and may be used as a pure compound, mixture, or derivative thereof.

In another embodiment, an injured skin model is provided by changes in the molar ratio of the lipid contents. A decrease (20% to 50%) in ceramide EOS, an increase (80% to 50%) of ceramide AP, a 10% to 15% decrease in the long chain free fatty acids (behenic acid:lignoceric acid: hexacosanoic acid) and a 10% to 15% increase in myristic acid.

In an embodiment, the thickness of the supported lipid bilayer mimicking healthy skin ranges from 10 nm - 16 nm and 5 nm - 7 nm (long and short lamellar periodicity phases) being 10% - 15% less in injured skin model.

## Assembly of supported lipid bilayer on polymeric scaffold

### Spin-coating assembly method

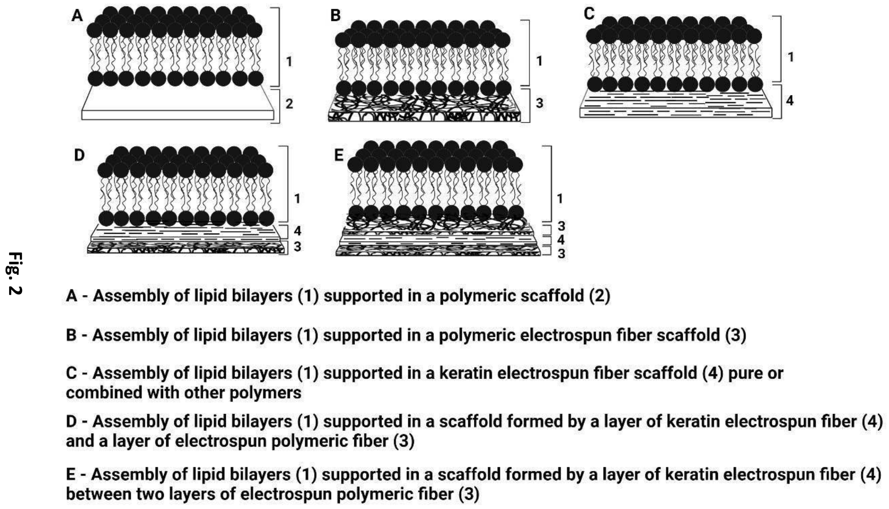

In an embodiment, the supported lipid bilayer is assembled on the polymeric scaffold of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof (Fig. 2A). Cholesterol (from 0% up to 35%) and phosphatidylcholine or its diacyl derivatives (from 65% up to 100%): from C(12)to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 μL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of polyvinyl alcohol, polylactic-co-glycolic acid, polylactic acid, polycaprolactone, polyethylene oxide, polyvinylpyrrolidone; poly(methyl methacrylate), ethyl cellulose or composites thereof (Fig. 2B). Cholesterol (from 0% up to 35%) and phosphatidylcholine or its diacyl derivatives (from 65% up to 100%): from C(12)to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 μL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayers are assembled on electrospun fibers of keratin and keratin derivatives from different sources (such as extracted from woven and non-woven sources) (Fig. 2C). Cholesterol (from 0% up to 35%) and phosphatidylcholine or its diacyl derivatives (from 65% up to 100%): from C(12)to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 μL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment the supported lipid bilayer is assembled on electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose (Fig. 2C). Cholesterol (from 0% up to 35%) and phosphatidylcholine or its diacyl derivatives (from 65% up to 100%): from C(12)to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 μL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose (Fig. 2D). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose (Fig. 2E). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In an embodiment, the supported lipid bilayer is assembled on the polymeric scaffold of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of polyvinyl alcohol, polylactic-co-glycolic acid, polylactic acid, polycaprolactone, polyethylene oxide, polyvinylpyrrolidone; poly(methyl methacrylate), ethyl cellulose or composites thereof. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin and keratin derivatives from different sources (such as extracted from woven and non-woven sources). A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 $\mu$L of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 $\mu$L of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 $\mu$L of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In an embodiment, the supported lipid bilayer is assembled on the polymeric scaffold of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of polyvinyl alcohol, polylactic-co-glycolic acid, polylactic acid, polycaprolactone, polyethylene oxide, polyvinylpyrrolidone; poly(methyl methacrylate), ethyl cellulose or composites thereof. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin and keratin derivatives from different sources (such as extracted from woven and non-woven sources). A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, two sequential depositions of 200 µL of multilamellar vesicles are spin-coated at 0.3 speed and intercalated by 20 s of spinning. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

**Extrusion assembly method**

In an embodiment, the supported lipid bilayer is assembled on the polymeric scaffold of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof (Fig. 2A). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 µL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of polyvinyl alcohol, polylactic-co-glycolic acid, polylactic acid, polycaprolactone, polyethylene oxide, polyvinylpyrrolidone; poly(methyl methacrylate), ethyl cellulose or composites thereof (Fig. 2B). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin and keratin derivatives from different sources (such as extracted from woven and non-woven sources) (Fig. 2C). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose (Fig. 2C). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose (Fig. 2D). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose (Fig. 2E). Cholesterol (from 0% up to 35%) and phosphatidylcholine or its diacyl derivatives (from 65% up to 100%): from C(12) to C(24) fatty acyl substituted glycero-3-phosphocholines are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In an embodiment, the supported lipid bilayer is assembled on the polymeric scaffold of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of $(3:16:12:1:1) \pm (0.3:1.7:1.3:0.1:0.5)$) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of polyvinyl alcohol, polylactic-co-glycolic acid, polylactic acid, polycaprolactone, polyethylene oxide, polyvinylpyrrolidone; poly(methyl methacrylate), ethyl cellulose or composites thereof. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of $(3:16:12:1:1) \pm (0.3:1.7:1.3:0.1:0.5)$) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin and keratin derivatives from different sources (such as extracted from woven and non-woven sources). A molar ratio of 23:10:33:33 of ceramide AP:ceramide EOS:cholesterol: free fatty acids (arachidonic acid:behenic acid:lignoceric acid: hexacosanoic acid:myristic acid in molar ratios of $(3:16:12:1:1) \pm (0.3:1.7:1.3:0.1:0.5)$) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of $(3:16:12:1:1) \pm (0.3:1.7:1.3:0.1:0.5)$) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 $\mu$L of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 $\mu$L of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose. A molar ratio of 23:10:33:33 of ceramide AP:ceramide EOS:cholesterol: free fatty acids (arachidonic acid:behenic acid:lignoceric acid: hexacosanoic acid:myristic acid in molar ratios of (3:16:12:1:1) $\pm$ (0.3:1.7:1.3:0.1:0.5)) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 $\mu$L of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In an embodiment, the supported lipid bilayer is assembled on the polymeric scaffold of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones such as hydrophilic polyethersulfone, or polycarbonate, or composites thereof. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 $\mu$L of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of polyvinyl alcohol, polylactic-co-glycolic acid, polylactic acid, polycaprolactone, polyethylene oxide, polyvinylpyrrolidone; poly(methyl methacrylate), ethyl cellulose or composites thereof. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin and keratin derivatives from different sources (such as extracted from woven and non-woven sources). A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on two polymeric layers. A first polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose; followed by a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone, poly(methyl methacrylate), ethyl cellulose. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

In another embodiment, the supported lipid bilayer is assembled on three polymeric layers. The first and the third polymeric layer of electrospun fibers of the following polymers or their mixtures: polyvinyl alcohol, polylactic-co-glycolic acid, polylactic acid, polycaprolactone, polyethylene oxide, polyvinylpyrrolidone, poly(methyl methacrylate), ethyl cellulose and in the middle a second polymeric layer of electrospun fibers of keratin or keratin derivatives from different sources (such as extracted from woven and non-woven sources) from 1% up to 50% combined with any of the following polymers (from 50% up to 99%) or their mixtures: polyvinyl alcohol, polylactic-co-glycolic acid, polylactic acid, polycaprolactone, polyethylene oxide, polyvinylpyrrolidone; poly(methyl methacrylate), ethyl cellulose. A molar ratio of 25 to 31:8 to 2:33:33 of ceramide AP:ceramide EOS:cholesterol: free fatty acids (arachidonic acid:behenic acid:lignoceric acid: hexacosanoic acid:myristic acid in molar ratios of (3:15 to 14:11.5 to 11:0.5 to 0:4 to 6) are first dissolved in chloroform and then evaporated under a stream of nitrogen, resulting in a thin lipid film. The dried lipid film is hydrated in a water/ethanol (10% v/v) mixture, and the resultant suspension is submitted to 2 to 10 cycles of vortexing and heated at temperature above the lipid phase transition to form multilamellar vesicles. Subsequently, 400 μL of the multilamellar vesicles are deposited through extrusion at room temperature. After deposition, an incubation period of 10 min at temperature above the lipid phase transition is used to promote the vesicle disruption through water and ethanol evaporation and consequent formation of planar supported lipid bilayer. Subsequently, the supported lipid bilayer is subjected to alternate cycles of submersion in liquid nitrogen, incubation for 30 min at temperature above the lipid phase transition and submersion in liquid nitrogen. The prepared supported lipid bilayer is stored in the fridge until use.

## Applications of the disclosure

An aspect of the present disclosure relates to its compatibility with high throughput screening (HTS) assays like microfluidic constructions, conventional Franz diffusion cells and other permeability assay designs, and is specifically made to be utilized in research of skin permeability and membrane interaction studies.

Another important aspect of the present disclosure is the possibility of replacing/removing the bioinspired skin model after permeation assays so that molecular interactions and biophysical changes in *stratum corneum* intercellular lipid matrix can be analysed, adding a new dimension to skin model evaluation.

In a further embodiment, the bioinspired skin model of the present disclosure can be integrated into microfluidic chips which have advantages of mimicking skin laminar flow to boost throughput and reduce cost, cut human intervention and errors, increase portability and automation setting a new standard for skin permeation studies.

An aspect of the present disclosure relates to its use to test skin permeability and analyse the molecular interactions and biophysical changes occurring at the skin model of different compounds, free or included in formulations.

In a further embodiment, the compounds tested can belong to different classes: drugs, drug candidates, bioactive ingredients, cosmetic ingredients, nutraceutics, cosmeceutics, natural compounds.

In another embodiment, the bioinspired skin model of the present disclosure can mimic cell-free *stratum corneum* lipid matrix models of injured skin conditions (e.g. atopic dermatitis), enabling researchers to examine how skin conditions affect barrier function and permeability. By including injured skin models of conditions like atopic dermatitis, which affect a large portion of the population, the present disclosure can be used to tailor treatments and formulations to specific patient profiles. This approach follows personalized medicine, which fits treatments to individual needs.

An aspect of the present disclosure relates to an artificial skin model comprising:

a polymeric support layer;
a lipid bilayer in the top of the polymeric support layer wherein the polymeric support layer supports the lipid bilayer, forming a multilayer structure;
wherein the polymeric support layer has a porosity ratio between 20% - 95% with a pore size inferior to 650 nm;
wherein the polymeric support layer is a protein-polymer composite;
wherein polymeric support layer is a polymeric scaffold.

In an embodiment, the polymeric support layer may comprise a plurality of sublayers and wherein the sublayers may be bound to each other.

In an embodiment, the polymeric support layer may be a polymeric scaffold.

In an embodiment, the thickness of the polymeric scaffold may range from 10 μm - 400 μm; preferably 10 μm - 100 μm; more preferably 10 μm - 60 μm.

In an embodiment, the polymeric support layer comprises a plurality of sublayers and wherein the sublayers are bound to each other.

In an embodiment, the polymer of the polymeric layer may be selected from a list consisting of cellulose, cellulose acetate, cellulose nitrate, cellulose hydrate, hydrophilic mixed cellulose esters, polyamides, polyethylene, polypropylene, polyurethane, polyethylenterephthalate, polyvinylfluoride, polyvinylchloride, polyvinylidendifluoride, polytetrafluoroethylen, polysulfones (such as, for example, hydrophilic polyethersulfone), polycarbonate, or combinations thereof.

In an embodiment, the polymeric scaffold may be obtained by electrospinning.

In an embodiment, the polymeric scaffold may comprise a mesh/bundle of fibers.

In an embodiment, the material of the polymeric scaffold fibers may be selected from a list consisting of: polyvinyl alcohol, polylactic-co-glycolic acid, polylactic acid, polycaprolactone, polyethylene oxide, polyvinylpyrrolidone, poly(methyl methacrylate), ethyl cellulose, or combinations thereof.

In an embodiment, the polymeric scaffold may comprise at least a protein; preferably a protein-polymer composite.

In an embodiment, the protein may be keratin, or a keratin derivative extracted from woven and non-woven sources.

In an embodiment, the plurality of sublayers may comprise a polymeric scaffold layer and a protein-polymer composite scaffold layer.

In an embodiment, the protein-polymer composite may comprise 1% - 50% ($w/w_{protein-polymer}$) of a protein and 50% - 99% ($w/w_{protein-polymer}$) of a polymer.

In an embodiment, the protein-polymer composite may comprise a protein combined with a polymer selected from a list consisting of: polyvinyl alcohol, polylactic-co-glycolic acid, polylactic acid, polycaprolactone, polyethylene oxide, polyvinylpyrrolidone, poly(methyl methacrylate), ethyl cellulose, or combinations thereof.

In certain embodiments, the disclosed artificial skin model comprises distinct lipid domains arranged to simulate the lamellar organization of the stratum corneum. In a further embodiment, the model includes: a first region comprising a lipid bilayer arrangement that mimics the long periodicity phase (LPP), exhibiting an extended lamellar spacing and a high degree of order; and a second region comprising a lipid bilayer arrangement that mimics the short periodicity phase (SPP), exhibiting a more compact lamellar organization.

In an embodiment, the lipid bilayer long periodicity phase thickness may range from 10 nm - 16 nm; preferably 9 nm - 15 nm; more preferably 8 nm - 14 nm as measured by atomic force microscopy or small angle X-ray scattering.

In an embodiment, the lipid bilayer short periodicity phase thickness may range from 5 nm - 7 nm; preferably 4 nm - 7 nm; as measured by atomic force microscopy or small angle X-ray scattering.

As used herein, the term "lipid bilayer long periodicity phase" (LPP) refers to a highly ordered lamellar arrangement of lipids characterized by an extended repeat distance between adjacent bilayers. The LPP exhibits a multi-lamellar structure with a relatively greater spacing and organization compared to the short periodicity phase. The LPP contributes to enhanced barrier strength and reduced permeability of the lipid matrix.

The term "lipid bilayer short periodicity phase" (SPP) designates a lamellar lipid arrangement exhibiting a more compact repeat distance between successive bilayers. The SPP represents a tighter and less extended organization compared to the long periodicity phase. This phase contributes to supplementary cohesion and flexibility within the lipid matrix. The coexistence of the SPP and LPP phases supports the structural integrity and dynamic function of the barrier layer.

In an embodiment, the lipid bilayer may comprise phosphatidylcholine; or phosphatidylcholine diacyl derivative; or a mixture of: cholesterol and phosphatidylcholine; or cholesterol and phosphatidylcholine diacyl derivative; or cholesterol, phosphatidylcholine and phosphatidylcholine diacyl derivative.

In an embodiment, the lipid bilayer comprises at least a lipid selected from the list consisting of: cholesterol, phosphatidylcholine, phosphatidylcholine diacyl derivatives, acylceramides, and a free fatty acid combination, or mixtures thereof; and/or the lipid bilayer comprises phosphatidylcholine; or phosphatidylcholine diacyl derivative; or a mixture of: cholesterol and phosphatidylcholine; or cholesterol and phosphatidylcholine diacyl derivative; or cholesterol, phosphatidylcholine and phosphatidylcholine diacyl derivative.

In an embodiment, the artificial skin model of the present disclosure may comprise 0.001 - 35% ($w/w_{lipid\ bilayer}$) of cholesterol; preferably 1% ($w/w_{lipid\ bilayer}$) - 35% ($w/w_{lipid\ bilayer}$) of cholesterol; more preferably 5 % ($w/w_{lipid\ bilayer}$) - 33 % ($w/w_{lipid\ bilayer}$) of cholesterol.

In an embodiment, the artificial skin model of the present disclosure may comprise 65% - 100% ($w/w_{lipid\ bilayer}$) of phosphatidylcholine or phosphatidylcholine diacyl derivatives; preferably 67% - 95% ($w/w_{lipid\ bilayer}$) phosphatidylcholine or phosphatidylcholine diacyl derivatives.

In an embodiment, the phosphatidylcholine diacyl derivatives may be C(12) - C(24) fatty acyl substituted glycero-3-phosphocholines derivatives; preferably C(14) - C(20).

In an embodiment, the lipid bilayer may comprise a mixture of an acylceramide combination, cholesterol, and at least two free fatty acids.

In an embodiment, the acylceramide may be selected from a list consisting of: ceramide AP, ceramide EOS, or combinations thereof.

In an embodiment, the fatty acid may be selected from a list consisting of: arachidonic acid, behenic acid, lignoceric acid, hexacosanoic acid, myristic acid, or combinations thereof; preferably arachidonic acid, behenic acid, lignoceric acid, hexacosanoic acid, and myristic acid.

In an embodiment, the molar ratio between ceramide AP:ceramide EOS:cholesterol:arachidonic acid:behenic acid:lignoceric acid:hexacosanoic acid:myristic acid may range from 23:2:32:2:13:10:0:0.4 to 35:10:34:4:18:14:1.2:4; preferably 23:2:33:2.7:13.5:10.7:0:0.5 to 35:10:33:3.3:17.7:13.3:1.1:4; more preferably 23:2:33:3:16:12:1:1 to 35:10:33:3:16:12:1:1.

In an embodiment, the molar ratio between ceramide AP:ceramide EOS:cholesterol:arachidonic acid:behenic acid:lignoceric acid:hexacosanoic acid:myristic acid may range from 23:10:33:3.3:17.7:10.6:0.9:0.5 to 23:10:33:2.7:14.3:13.4:1.1:1.5; more preferably 23:10:33:3:16:12:1:1.

In an embodiment, the molar ratio of ceramide AP:ceramide EOS: cholesterol: arachidonic acid: behenic acid: lignoceric acid: hexacosanoic acid: myristic acid may be 23:10:33:3:16:12:1:1.

In an embodiment, the artificial skin model of the present disclosure may mimic an artificial healthy human skin model.

In an embodiment, the molar ratio between ceramide AP: ceramide EOS: cholesterol: arachidonic acid:behenic acid:lignoceric acid:hexacosanoic acid:myristic acid may be 28:5:3:15:11.5:0.5:2; preferably 28:5:3:13.5:11:0:4; more preferably 28:5:3:14.25:11.25:0.25:3.

In an embodiment, the molar ratio between ceramide AP: ceramide EOS: cholesterol: arachidonic acid:behenic acid:lignoceric acid: hexacosanoic acid:myristic acid may range from 25:8:33:3:15:11.5:0.5:4 to 31:2:33:3:14:11:0:6, more preferably 28:5:33:3:14.5:11.25:0.25:5.

In an embodiment, the artificial skin model of the present disclosure may mimic an artificial atopic dermatitis human skin model.

In an embodiment, the molar ratio of ceramide EOS in the artificial atopic dermatitis human skin model may have a decrease of 20% - 50% as compared to the artificial healthy human skin model.

In an embodiment, the molar ratio of ceramide AP in the artificial atopic dermatitis human skin model may have an increase of 80% - 50% as compared to artificial healthy human skin model.

In an embodiment, the molar ratio of long chain free fatty acid in the artificial atopic dermatitis human skin model may have a decrease of 10% - 15% as compared to artificial healthy human skin model.

In an embodiment, the molar ratio of myristic acid in the artificial atopic dermatitis human skin model may have a decrease of 10% - 15% as compared to artificial healthy human skin model.

An aspect of the present disclosure relates to its use in the screening of a pharmaceutical and/or a cosmetical active ingredient.

Another aspect of the present disclosure relates to a kit for use in the screening of pharmaceutical or cosmetical active ingredients comprising the artificial skin model described in the present disclosure.

An aspect of the present disclosure relates to its use as a screener of a pharmaceutical and/or a cosmetical active ingredient; or as artificial healthy human skin model; or as an atopic dermatitis human skin model.

An aspect of the present disclosure relates to a method for producing the lipid bilayer described in the present disclosure, comprising the steps:

providing a lipid mixture;
dissolving the lipid mixture in an organic solvent;
evaporating the organic solvent forming a thin lipid film;
drying the thin lipid film to obtain a dried thin lipid film;
hydrating the dried thin lipid film in water/ethanol mixture to obtain a suspension (10% v/v); vortexing the suspension for 2-10 cycles;
heating the suspension at temperature above the lipid phase transition to obtain the lipid bilayer in the form of multilamellar vesicles.

Another aspect of the present disclosure relates to a method for obtaining the artificial skin model of the present disclosure, comprising the steps:

providing the polymeric scaffold and the lipid bilayer described in the present disclosure;
spin-coating the lipid bilayer in the form of multilamellar vesicles at 0.3 speed and intercalated by 20 s of spinning;
incubating the spin-coated lipid bilayer in the form of multilamellar vesicles for 10 min at temperature above the lipid phase transition to promote the vesicle disruption through water and ethanol evaporation;
forming a planar supported lipid bilayer;
assembling the lipid bilayer on the polymeric support layer;
obtaining the artificial skin model.

Even another aspect of the present disclosure relates to a method for obtaining the artificial skin model of the present disclosure, comprising the steps:

providing the polymeric scaffold and the lipid bilayer described in the present disclosure;
depositing the lipid bilayer on the polymeric support layer via extrusion of the lipid bilayer in the form of multilamellar vesicles;
obtaining the artificial skin model.

## BRIEF DESCRIPTION OF THE DRAWINGS

The following figures provide preferred embodiments for illustrating the disclosure and should not be seen as limiting the scope of invention.

**Figure 1-** Schematic illustration of intercellular lipid matrix.

**Figure 2 -** Assembly of supported lipid bilayers on polymeric scaffolds.

**Figure 3** - Calorimetric and morphological characterization of skin model, showing the coexistence of different lipid domains with lipid phases and lamellar periodicities similar to the native skin.

**Figure 4** - Morphological micrographs of polymeric scaffolds before and after skin model deposition at different magnifications.

**Figure 5** - Stability studies of the skin model on polycarbonate and cellulose polymeric scaffolds through water contact angle measurements as function of storage time in the freezer.

**Figure 6** - Cumulative amount of model compounds (caffeine, diclofenac and testosterone) permeation profiles on acceptor compartment through skin model mounted on a typical Franz cell apparatus. Results are reported as mean of 3-5 parallel experiments $\pm$ range.

**Figure 7** - Cumulative amount of model compounds (caffeine, diclofenac and testosterone) permeation profiles on acceptor compartment through DPPC and DPPC:Ch(2:1) models mounted on a typical Franz cell apparatus. Results are reported as mean of 3-5 parallel experiments $\pm$ range.

**Figure 8** - Values of water loss rate (WLR) through the different lipid models (skin, DPPC and DPPC:Chol(2:1)) on polycarbonate and cellulose scaffold at 24 h, and respective values of occlusive effect (%OE).

## DETAILED DESCRIPTION

The present disclosure relates to an artificial skin model that mimics the intercellular lipid matrix of the *stratum corneum* in healthy or injured skin, including the short and long lamellar periodicity phases that are thought to be essential to the barrier function of the skin. In particular, the present disclosure relates to simple and cost-effective methods and processes for HTS of skin permeation assays and membrane interaction studies, particularly useful in the pharmaceutical, cosmetic, and chemical industries for the evaluation of topical formulations, transdermal delivery systems, and other skin-related treatments.

As used herein, the term "artificial skin model" refers to a synthetic or reconstructed multilayer system designed to reproduce selected structural and functional features of the stratum corneum. In certain embodiments, the artificial model comprises a region that mimics the long periodicity phase (LPP) of the lipid bilayer and a region that mimics the short periodicity phase (SPP). Each region may reproduce the respective lipid organization, composition, and molecular packing characteristics observed in natural skin, thereby enabling the study or modulation of barrier-related properties in a controlled environment.

Cell-free lipid models offer better reproducibility, ease of handling, and cost-effectiveness compared to cellular skin models. More representative skin protein-to-lipid ratio, and more representative lipid matrix of the stratum corneum (mimicking the short and long lamellar periodicity phases that are essential to the barrier function of the skin) enhancing the model's physiological relevance.

Versatile skin models representing healthy skin and injured skin conditions offering insights into the efficacy and safety of skin care products tailored to individual patient profiles and following the principles of personalized medicine.

In an aspect of the present disclosure, the bioinspired skin models can be inserted in several constructs (e.g. classical Franz diffusion cells or microfluidic diffusion chips) and removed from them enabling post-permeation analysis of molecular interactions and biophysical changes occurring at the skin level.

In another aspect of the present disclosure, the bioinspired skin models can be produced in large quantities and stored for extended periods of time before use, allowing for the shipment of either the models or the entire permeation construct.

### Examples

### Example 1

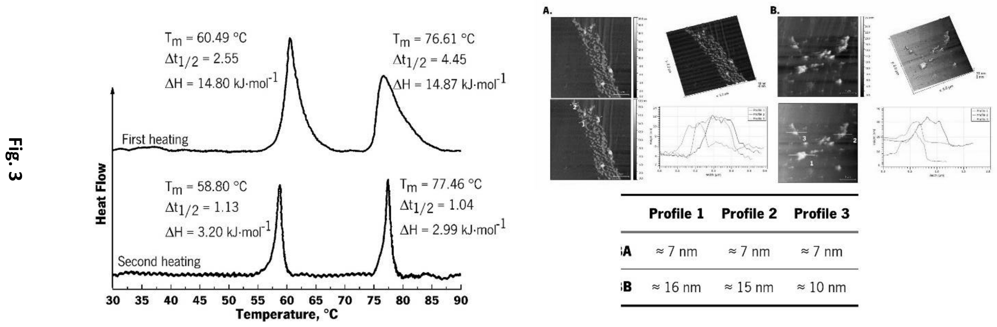

In an embodiment for better results, two different polymeric scaffolds, cellulose and polycarbonate were considered to support the bioinspired skin model (referred as skin model in **Fig. 3, 4, 5, 6,** and **8)** composed of: 23% of ceramide EOS, 10 % of ceramide AP, 33 % of cholesterol, 3 % of arachidonic acid, 16 % of behenic acid, 13 % of lignoceric acid, 1.3 % of hexacosanoic acid and 0.7 % of myristic acid. The deposition protocols were dependent on the scaffold. Skin model (400 $\mu$L of multilamellar vesicles) was deposited on polycarbonate scaffold via extrusion at room temperature or on cellulose scaffold by two sequential depositions (200 $\mu$L of multilamellar vesicles) via spin-coating.

**Fig. 3** shows various embodiments of the present disclosure, including the skin model before and after deposition on polymeric scaffolds. Both calorimetric and morphological analysis revealed the coexistence of various lipid domains. The skin model, like the native skin [12-14], has a multilamellar structure with short-phase and long-phase periodicities, with repeated distances of $\approx$7 nm and 10-16 nm, respectively.

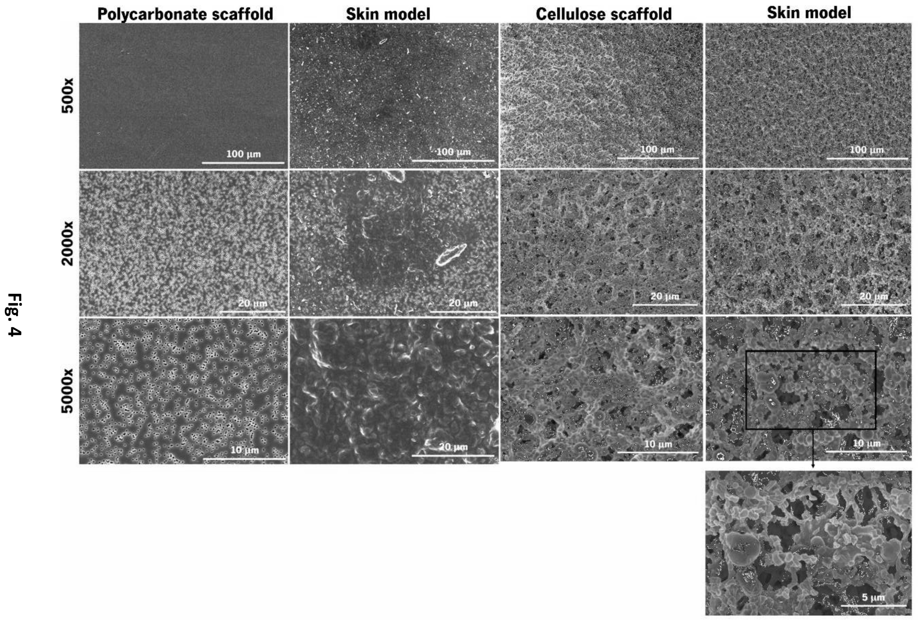

**Fig. 4** shows the morphological characterization of the disclosure on two embodiments of the present disclosure, namely, two different polymeric scaffolds. While the skin model over the polycarbonate scaffold formed a flat surface, the structure in cellulose scaffold presents a 3D distribution in the z-axis, resulting in a 3D mesh-like structure.

This example illustrates how the present disclosure replicates the physiological characteristics of native skin.

### Example 2

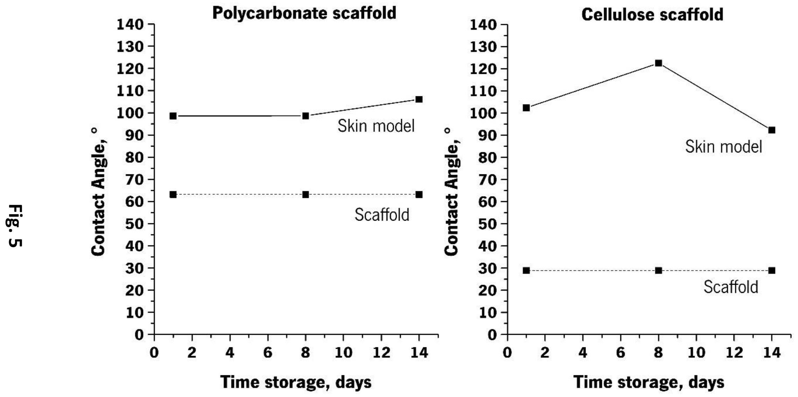

In an embodiment for better results, after deposition of skin model on polymeric scaffolds, water contact angle measurements were carried out not only to confirm the deposition at the day 0 of the preparation, but also to assess the stability of the coverage over time storage in the freezer. **Fig. 5** shows how the surface properties of polymeric scaffolds change after skin model deposition in all the tested examples. The stability of the supported lipid bilayers is practically independent on the polymeric scaffold stored in the freezer for at least 14 days.

### Example 3

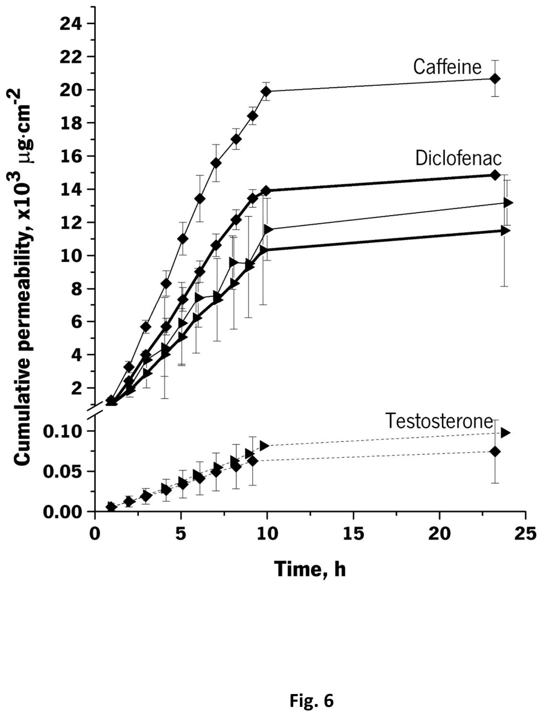

In an embodiment for better results, the permeabilities of three different model compounds (diclofenac, caffeine and testosterone) were measured through the skin model **(Fig. 6)** prepared in two different polymeric scaffolds, as described in Example 1. The skin model was mounted on a Franz cell apparatus with a permeation surface of 0.64 cm$^2$, a maximum volume in the upper compartment of 0.8 mL and a fixed volume in the bottom compartment of 5 mL. The permeability experiment was carried out over ~24 h at 32 °C. The bottom compartment was filled with 5 mL of acetate buffer (pH 5.5) and the skin model was placed between upper and bottom compartment at time zero. Every hour, 0.2 mL of the solution from the bottom compartment were withdrawn and compound concentration was quantified by UV-Vis spectrophotometry. The removed volume was replaced with an equal volume of fresh buffer. The results obtained showed that the disclosure was successful in ranking the model compounds according to its skin permeability in the same order as literature data [15-17]. These results are important to prove the reliability of the disclosure, since it can distinguish the permeation profiles of model compounds with different chemical characteristics.

### Example 4

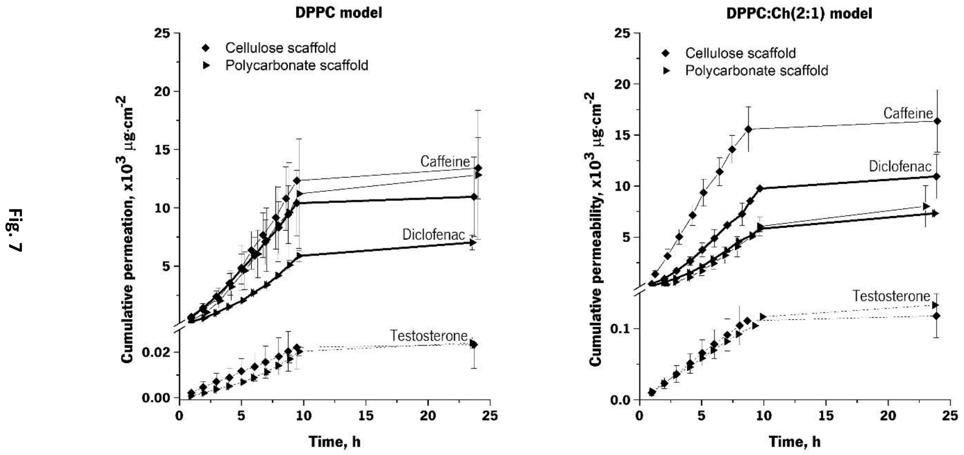

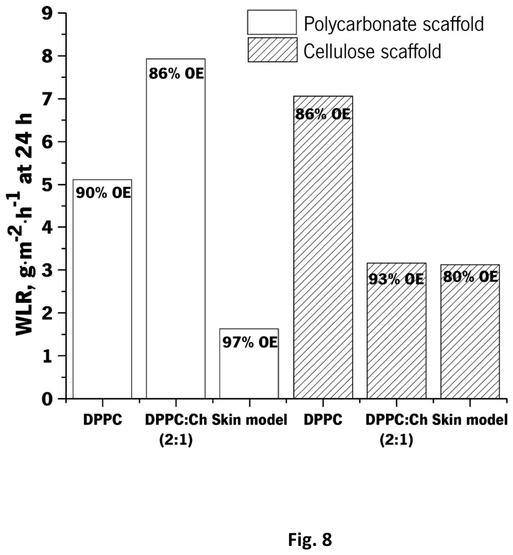

In an embodiment for better results, the permeabilities of the model compounds (diclofenac, caffeine and testosterone) were determined through other two different lipid models containing 100% of phosphatidylcholine (referred as DPPC in Fig. 7 and 8) or phosphatidylcholine and 33% of cholesterol (referred as DPPC:Ch (2:1) in Fig. 7 and 8) employing the same methods described in Example 1 **(Fig. 7).**

**Fig. 7** compares the amounts of each compound that pass through the model barriers over time. In both lipid models, the permeability differences between the three compounds remain, with caffeine being the most permeable and testosterone being the least permeable. These results are consistent with literature [15-17].

This example demonstrates not only that the disclosure can be used with compounds of varying lipophilicity, but also that the lipid models can be tuned to mimic specific physiological conditions while maintaining reliability and permeability determination ability.

### Example 5

In an embodiment for better results, water loss rate experiments were carried out using lipid models supported on two different polymeric scaffolds (cellulose and polycarbonate) for 24 h, which is the duration of a typical permeability experiment. The values of both water loss rate and occlusive effect obtained **(Fig. 8)** at 24 h reflect the ability of lipid models to maintain their integrity under typical permeability experimental conditions. The values obtained from this example are within the range described in literature for healthy human skin [18-20].

This example is relevant not only to demonstrate the reliability of the skin model described in the disclosure, but also to ensure that the barrier integrity remains intact throughout the typical permeability experiment time range of 24 h. Furthermore, the results indicate model cohesion, with typical occlusive values for semipermeable barriers such as native cell lipid bilayers. This example proves that the amount of compound detected in the receptor chamber is due to free diffusion through the lipid models, rather than poor barrier properties.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## References

1. Lee, D.H., et al., Advancements in Skin-Mediated Drug Delivery: Mechanisms, Techniques, and Applications. Adv Healthc Mater, 2024. 13(7): p. e2302375.

2. Fernandes, E., et al., A Stratum corneum lipid model as a platform for biophysical profiling of bioactive chemical interactions at the skin level. Journal of Molecular Liquids, 2024. 400: p. 124513.

3. Lopes, C.M., et al., Chapter 14 - Lipid-based colloidal carriers for topical application of antiviral drugs, in Design of Nanostructures for Versatile Therapeutic Applications, A.M. Grumezescu, Editor. 2018, William Andrew Publishing. p. 565-622.

4. van Smeden, J. and J.A. Bouwstra, Stratum Corneum Lipids: Their Role for the Skin Barrier Function in Healthy Subjects and Atopic Dermatitis Patients. Curr Probl Dermatol, 2016. 49: p. 8-26.

5. Haq, A., et al., Strat-M(R) synthetic membrane: Permeability comparison to human cadaver skin. Int J Pharm, 2018. 547(1-2): p. 432-437.

6. Neupane, R., et al., Alternatives to Biological Skin in Permeation Studies: Current Trends and Possibilities. Pharmaceutics, 2020. 12(2).

7. Silva, R.J. and S. Tamburic, A State-of-the-Art Review on the Alternatives to Animal Testing for the Safety Assessment of Cosmetics. Cosmetics, 2022. 9(5): p. 90.

8. Henning, A., U.F. Schaefer, and D. Neumann, Potential pitfalls in skin permeation experiments: influence of experimental factors and subsequent data evaluation. Eur J Pharm Biopharm, 2009. 72(2): p. 324-31.

9. van Gele, M., et al., Three-dimensional skin models as tools for transdermal drug delivery: challenges and limitations. Expert Opinion on Drug Delivery, 2011. 8(6): p. 705-720.

10. Ponec, M., In vitro cultured human skin cells as alternatives to animals for skin irritancy screening. Int J Cosmet Sci, 1992. 14(6): p. 245-64.

11. Costa, T. et al., Polymeric Electrospun Fibrous Dressings for Topical Co-delivery of Acyclovir and Omega-3 Fatty Acids. Frontiers in Bioengineering and Biotechnology, 2019. 7.

12. Bouwstra, J.A., G.S. Gooris, J.A. van der Speck, and W. Bras, Structural investigations of human stratum corneum by small-angle X-ray scattering. J. Invest. Dermatol., 1991. 97(6): p. 1005-1012.

13. Schmitt, T., S. Lange, S. Sonnenberger, B. Dobner, et al., The long periodicity phase (LPP) controversy part I: The influence of a natural-like ratio of the CER[EOS] analogue [EOS]-br in a CER[NP]/[AP] based stratum corneum modelling system: A neutron diffraction study. Biochim. Biophys. Acta, Biomembr., 2019. 1861(1): p. 306-315.

14. Hatta, I., N. Ohta, K. Inoue, and N. Yagi, Coexistence of two domains in intercellular lipid matrix of stratum corneum. Biochim. Biophys. Acta, 2006. 1758(11): p.1830-1836.

15. Dias, M. et al. , "Topical delivery of caffeine from some commercial formulations", International Journal of Pharmaceutics, vol. 182, pp. 41-47, 1999.

16. Degim, I. et al., "Skin permeability data: Anomalous results", International Journal of Pharmaceutics, vol. 170 pp. 129-133, 1998.

17. Kim, M. et al. , "Skin Permeation of Testosterone and its Ester Derivatives in Rats", Journal of Pharmacy and Pharmacology, vol. 52, pp. 369-375, 2010.

18. Firooz, A., H. Zartab, B. Sadr, L.N. Bagherpour, et al., Daytime Changes of Skin Biophysical Characteristics: A Study of Hydration, Transepidermal Water Loss, pH, Sebum, Elasticity, Erythema, and Color Index on Middle Eastern Skin. Indian J. Dermatol., 2016. 61(6): p. 700.

19. Green, M., N. Kashetsky, A. Feschuk, and H.I. Maibach, Transepidermal water loss (TEWL): Environment and pollution-A systematic review. Skin Health Dis., 2022. 2(2): p. e104.20. Kottner, J., A. Vogt, A. Lichterfeld, and U. Blume-Peytavi, Transepidermal Water Loss in Young and Aged Healthy Humans, in Textbook of Aging Skin, M.A. Farage, K.W. Miller, and H.I. Maibach, Editors. 2015, Springer Berlin, Heidelberg.

**Claims**

1. Artificial skin model comprising:

   a polymeric support layer;
   a lipid bilayer in the top of the polymeric support layer wherein the polymeric support layer supports the lipid bilayer;
   wherein the polymeric support layer has a porosity ratio between 20% - 95% with a pore size inferior to 650 nm;
   wherein the polymeric support layer is a protein-polymer composite;
   wherein the polymeric support layer is a polymeric scaffold.

2. Artificial skin model according to the previous claim, wherein the polymeric support layer comprises a plurality of sublayers and wherein the sublayers are bound to each other.

3. Artificial skin model according to any of the previous claims, wherein the protein-polymer composite comprises 1% - 50% ($w/w_{protein-polymer}$) of a protein and 50% - 99% ($w/w_{protein-polymer}$) of a polymer.

4. Artificial skin model according to the previous claim, wherein the protein is keratin, or a keratin derivative extracted from woven and non-woven sources.

5. Artificial skin model according to any of the previous claims 3-4, wherein the polymer is selected from a list consisting of: polyvinyl alcohol, polylactic-co-glycolic acid, polylactic acid, polycaprolactone, polyethylene oxide, polyvinylpyrrolidone; poly(methyl methacrylate), ethyl cellulose, or combinations thereof.

6. Artificial skin model according to any of the previous claims, wherein the lipid bilayer comprises at least a lipid selected from the list consisting of: cholesterol, phosphatidylcholine, phosphatidylcholine diacyl derivatives, acylceramides, and a free fatty acid combination, or mixtures thereof; and/or wherein the lipid bilayer comprises phosphatidylcholine; or phosphatidylcholine diacyl derivative; or a mixture of: cholesterol and phosphatidylcholine; or cholesterol and phosphatidylcholine diacyl derivative; or cholesterol, phosphatidylcholine and phosphatidylcholine diacyl derivative.

7. Artificial skin model according to any of the previous claims, comprising 0.001 - 35% ($w/w_{lipid\ bilayer}$) of cholesterol; preferably 1% ($w/w_{lipid\ bilayer}$) - 35% ($w/w_{lipid\ bilayer}$) of cholesterol; more preferably 5 % ($w/w_{lipid\ bilayer}$) - 33 % ($w/w_{lipid\ bilayer}$) of cholesterol; and/or comprising 65% - 100% ($w/w_{lipid\ bilayer}$) of phosphatidylcholine or phosphatidylcholine diacyl derivatives; preferably 67% - 95% ($w/w_{lipid\ bilayer}$) of phosphatidylcholine or phosphatidylcholine diacyl derivatives.

8. Artificial skin model according to any of the previous claims 6-7, wherein the phosphatidylcholine diacyl derivatives are C(12) - C(24) fatty acyl substituted glycero-3-phosphocholines derivatives; preferably C(14) - C(20).

9. Artificial skin model according to the previous claims 6, wherein the lipid bilayer comprises a mixture of an acylceramide combination, cholesterol, and at least two free fatty acids; preferably wherein the acylceramide combination is selected from a list consisting of: ceramide AP, ceramide EOS, or combinations thereof; and/or wherein the free fatty acid is selected from a list consisting of: arachidonic acid, behenic acid, lignoceric acid, hexacosanoic acid, myristic acid, or combinations thereof; preferably arachidonic acid, behenic acid, lignoceric acid, hexacosanoic acid, and myristic acid.

10. Artificial skin model according to any of the previous claims 6 or 9, wherein the molar ratio between ceramide AP:ceramide EOS:cholesterol:arachidonic acid:behenic acid:lignoceric acid:hexacosanoic acid:myristic acid ranges from 23:10:33:3.3:17.7:10.6:0.9:0.5 to 23:10:33:2.7:14.3:13.4:1.1:1.5; more preferably is 23:10:33:3:16:12:1:1; and/or the molar ratio between ceramide AP: ceramide EOS: cholesterol: arachidonic acid:behenic acid:lignoceric acid: hexacosanoic acid:myristic acid ranges from 25:8:33:3:15:11.5:0.5:4 to 31:2:33:3:14:11:0:6, more preferably is 28:5:33:3:14.5:11.25:0.25:5.

11. Artificial skin model according to any of the previous claims, wherein the lipid bilayer long periodicity phase thickness ranges from 10 nm - 16 nm; preferably 9 nm - 15 nm; more preferably 8 nm - 14 nm; and/or the lipid bilayer short periodicity phase thickness ranges from 5 nm - 7 nm; preferably 4 nm - 7 nm.

12. Artificial skin model according to any of the previous claims, wherein the thickness of the polymeric scaffold ranges from 10 $\mu$m - 400 $\mu$m; preferably 10 $\mu$m - 100 $\mu$m; more preferably 10 $\mu$m - 60 $\mu$m.

13. Artificial skin model according to any of the previous claims, for use in the screening of a pharmaceutical and/or a cosmetical active ingredient.

14. Kit for use in the screening of pharmaceutical or cosmetical active ingredient comprising an artificial skin model described in any of the previous claims.

15. Use of the artificial skin model according to any of the previous claims as a screener of a pharmaceutical and/or a cosmetical active ingredient; or as artificial healthy human skin model; or as an atopic dermatitis human skin model.

**Fig. 1**

A - Assembly of lipid bilayers (1) supported in a polymeric scaffold (2)

B - Assembly of lipid bilayers (1) supported in a polymeric electrospun fiber scaffold (3)

C - Assembly of lipid bilayers (1) supported in a keratin electrospun fiber scaffold (4) pure or combined with other polymers

D - Assembly of lipid bilayers (1) supported in a scaffold formed by a layer of keratin electrospun fiber (4) and a layer of electrospun polymeric fiber (3)

E - Assembly of lipid bilayers (1) supported in a scaffold formed by a layer of keratin electrospun fiber (4) between two layers of electrospun polymeric fiber (3)

Fig. 2

EP 4 737 559 A1

**Fig. 3**

$T_m = 60.49\ ^\circ C$
$\Delta t_{1/2} = 2.55$
$\Delta H = 14.80\ kJ\cdot mol^{-1}$

$T_m = 76.61\ ^\circ C$
$\Delta t_{1/2} = 4.45$
$\Delta H = 14.87\ kJ\cdot mol^{-1}$

First heating

$T_m = 58.80\ ^\circ C$
$\Delta t_{1/2} = 1.13$
$\Delta H = 3.20\ kJ\cdot mol^{-1}$

$T_m = 77.46\ ^\circ C$
$\Delta t_{1/2} = 1.04$
$\Delta H = 2.99\ kJ\cdot mol^{-1}$

Second heating

Heat Flow

Temperature, °C

30  35  40  45  50  55  60  65  70  75  80  85  90

A.

B.

| | Profile 1 | Profile 2 | Profile 3 |
|---|---|---|---|
| A | ≈ 7 nm | ≈ 7 nm | ≈ 7 nm |
| B | ≈ 16 nm | ≈ 15 nm | ≈ 10 nm |

**Fig. 4**

Fig. 5

**Fig. 6**

Fig. 7

**Fig. 8**

Europäisches
Patentamt

European
Patent Office

Office européen
des brevets

**EUROPEAN SEARCH REPORT**

Application Number

EP 25 20 9608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BALINT SINKÓ: "SkinPAMPA: A new method for fast prediction of skin penetration", EUROPEAN JOURNAL OF PHARMACEUTICAL SCIENCES- AUTHOR MANUSCRIPT ELSEVIER AMSTERDAM, NL, vol. 45, no. 5, 24 January 2012 (2012-01-24), pages 698-707, XP028464594, ISSN: 0928-0987, DOI: 10.1016/J.EJPS.2012.01.011 [retrieved on 2012-02-02] * Abstract *; page 698, paragraph 1 * page 699, right-hand column, paragraph 3 * * page 702; table 5 * * page 704 - page 705, paragraph 1 * ----- | 1-15 | INV. C12N5/071 G01N15/08 G01N33/50 C07K14/78 C12M1/12 |
| Y | JP 4 426329 B2 (CHUGAI PHARMACEUTICAL CO LTD) 3 March 2010 (2010-03-03) * paragraph [0044] - paragraph [0047]; figures 1,2,4; examples 1, 2 * ----- | 1-15 | |
| Y,D | FERNANDES EDUARDA ET AL: "A Stratum corneum lipid model as a platform for biophysical profiling of bioactive chemical interactions at the skin level", JOURNAL OF MOLECULAR LIQUIDS, ELSEVIER, AMSTERDAM, NL, vol. 400, 18 March 2024 (2024-03-18), XP087504642, ISSN: 0167-7322, DOI: 10.1016/J.MOLLIQ.2024.124513 [retrieved on 2024-03-18] * page 2 - page 3; figure 1 * ----- -/-- | 1-15 | TECHNICAL FIELDS SEARCHED (IPC) C12N G01N C12M C07K |

The present search report has been drawn up for all claims

| Place of search | Date of completion of the search | Examiner |
|---|---|---|
| Munich | 18 March 2026 | Heiduschat, Carola |

EPO FORM 1503 03.82 (P04C01)

Europäisches
Patentamt

European
Patent Office

Office européen
des brevets

## EUROPEAN SEARCH REPORT

**Application Number**

EP 25 20 9608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MATSUI TAKESHI ET AL: "Dissecting the formation, structure and barrier function of the stratum corneum", INTERNATIONAL IMMUNOLOGY, [Online] vol. 27, no. 6, 26 March 2015 (2015-03-26) , pages 269-280, XP093376029, GB ISSN: 0953-8178, DOI: 10.1093/intimm/dxv013 Retrieved from the Internet: URL:https://academic.oup.com/intimm/article-pdf/27/6/269/45027212/intimm_27_6_269.pdf> [retrieved on 2026-03-12] * Abstract *; page 269 * page 275; figure 2 * * page 276, right-hand column, last paragraph - page 277, left-hand column, paragraph 2 * | 1-15 | |
| A | SUGANO K ET AL: "optimized conditions of bio-mimetic artificial membrane permeation assay", 1 January 2001 (2001-01-01), INTERNATIONAL JOURNAL OF PHARMACEUTICS ELSEVIER, AMSTERDAM, NL, PAGE(S) 181 - 188, XP002259961, ISSN: 0378-5173 * page 183, right-hand column * * page 187, last paragraph * | 1-15 | TECHNICAL FIELDS SEARCHED (IPC) |
| A | EP 1 521 962 B1 (PION INC [US]) 23 December 2009 (2009-12-23) * paragraph [0001]; claims 1, 10-12 * | 1-15 | |

-/--

The present search report has been drawn up for all claims

| Place of search | Date of completion of the search | Examiner |
|---|---|---|
| Munich | 18 March 2026 | Heiduschat, Carola |

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JUNG SUNJUN ET AL: "Transformation of electrospun Keratin/PVA nanofiber membranes into multilayered 3D Scaffolds: Physiochemical studies and corneal implant applications", INTERNATIONAL JOURNAL OF PHARMACEUTICS ELSEVIER, AMSTERDAM, NL, vol. 610, 26 October 2021 (2021-10-26), XP086885554, ISSN: 0378-5173, DOI: 10.1016/J.IJPHARM.2021.121228 [retrieved on 2021-10-26] * page 2, left-hand column, last paragraph - page 3, left-hand column, paragraph f * * page 4; figure 1 * | 1-15 | |
| A | EP 3 615 096 B1 (UNIV TEMPLE [US]) 9 August 2023 (2023-08-09) * claims 1, 11-15 * | 1-15 | |
| | | | TECHNICAL FIELDS SEARCHED (IPC) |

The present search report has been drawn up for all claims

| Place of search | Date of completion of the search | Examiner |
|---|---|---|
| Munich | 18 March 2026 | Heiduschat, Carola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 9608

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2026

| Patent document cited in search report | | Publication date | Patent family member(s) | | Publication date |
|---|---|---|---|---|---|
| JP 4426329 | B2 | 03-03-2010 | JP | 4426329 B2 | 03-03-2010 |
| | | | JP | 2005221442 A | 18-08-2005 |
| EP 1521962 | B1 | 23-12-2009 | AT | E453119 T1 | 15-01-2010 |
| | | | EP | 1521962 A2 | 13-04-2005 |
| | | | US | 2003219716 A1 | 27-11-2003 |
| | | | WO | 03065037 A2 | 07-08-2003 |
| EP 3615096 | B1 | 09-08-2023 | CN | 110799221 A | 14-02-2020 |
| | | | EP | 3615096 A1 | 04-03-2020 |
| | | | IL | 270176 A | 31-12-2019 |
| | | | JP | 7281196 B2 | 25-05-2023 |
| | | | JP | 2020517382 A | 18-06-2020 |
| | | | KR | 20190141732 A | 24-12-2019 |
| | | | US | 2021046213 A1 | 18-02-2021 |
| | | | US | 2023364298 A1 | 16-11-2023 |
| | | | WO | 2018200776 A1 | 01-11-2018 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016078667 A

### Non-patent literature cited in the description

- **LEE, D.H et al.** Advancements in Skin-Mediated Drug Delivery: Mechanisms, Techniques, and Applications.. *Adv Healthc Mater*, 2024, vol. 13 (7), e2302375
- **FERNANDES, E. et al.** A Stratum corneum lipid model as a platform for biophysical profiling of bioactive chemical interactions at the skin level.. *Journal of Molecular Liquids*, 2024, vol. 400, 124513
- Lipid-based colloidal carriers for topical application of antiviral drugs. **LOPES, C.M. et al.** Design of Nanostructures for Versatile Therapeutic Applications. William Andrew Publishing, 2018, 565-622
- **VAN SMEDEN, J ; J.A. BOUWSTRA**. Stratum Corneum Lipids: Their Role for the Skin Barrier Function in Healthy Subjects and Atopic Dermatitis Patients.. *Curr Probl Dermatol*, 2016, vol. 49, 8-26
- **HAQ, A. et al.** Strat-M(R) synthetic membrane: Permeability comparison to human cadaver skin.. *Int J Pharm*, 2018, vol. 547 (1-2), 432-437
- **NEUPANE, R. et al.** Alternatives to Biological Skin in Permeation Studies: Current Trends and Possibilities.. *Pharmaceutics*, 2020, vol. 12 (2)
- **SILVA, R.J. ; S. TAMBURIC**. A State-of-the-Art Review on the Alternatives to Animal Testing for the Safety Assessment of Cosmetics.. *Cosmetics*, 2022, vol. 9 (5), 90
- **HENNING, A ; U.F. SCHAEFER ; D. NEUMANN**. Potential pitfalls in skin permeation experiments: influence of experimental factors and subsequent data evaluation.. *Eur J Pharm Biopharm*, 2009, vol. 72 (2), 324-31
- **VAN GELE, M et al.** Three-dimensional skin models as tools for transdermal drug delivery: challenges and limitations.. *Expert Opinion on Drug Delivery*, 2011, vol. 8 (6), 705-720
- **PONEC, M**. In vitro cultured human skin cells as alternatives to animals for skin irritancy screening. *Int J Cosmet Sci*, 1992, vol. 14 (6), 245-64

- **COSTA, T. et al.** Polymeric Electrospun Fibrous Dressings for Topical Co-delivery of Acyclovir and Omega-3 Fatty Acids.. *Frontiers in Bioengineering and Biotechnology*, 2019, vol. 7
- **BOUWSTRA, J.A. ; G.S. GOORIS ; J.A. VAN DER SPECK ; W. BRAS**. Structural investigations of human stratum corneum by small-angle X-ray scattering. *J. Invest. Dermatol.*, 1991, vol. 97 (6), 1005-1012
- **SCHMITT, T ; S. LANGE ; S. SONNENBERGER ; B. DOBNER et al.** The long periodicity phase (LPP) controversy part I: The influence of a natural-like ratio of the CER[EOS] analogue [EOS]-br in a CER [NP]/[AP] based stratum corneum modelling system: A neutron diffraction study.. *Biochim. Biophys. Acta, Biomembr*, 2019, vol. 1861 (1), 306-315
- **HATTA, I. ; N. OHTA ; K. INOUE ; N. YAGI**. Coexistence of two domains in intercellular lipid matrix of stratum corneum.. *Biochim. Biophys. Acta*, 2006, vol. 1758 (11), 1830-1836
- **DIAS, M. et al.** Topical delivery of caffeine from some commercial formulations. *International Journal of Pharmaceutics*, 1999, vol. 182, 41-47
- **DEGIM, I. et al.** Skin permeability data: Anomalous results. *International Journal of Pharmaceutics*, 1998, vol. 170, 129-133
- **KIM, M. et al.** Skin Permeation of Testosterone and its Ester Derivatives in Rats. *Journal of Pharmacy and Pharmacology*, 2010, vol. 52, 369-375
- **FIROOZ, A. ; H. ZARTAB ; B. SADR ; L.N. BAGHERPOUR et al.** Daytime Changes of Skin Biophysical Characteristics: A Study of Hydration, Transepidermal Water Loss, pH, Sebum, Elasticity, Erythema, and Color Index on Middle Eastern Skin.. *Indian J. Dermatol.*, 2016, vol. 61 (6), 700
- **GREEN, M. ; N. KASHETSKY ; A. FESCHUK ; H.I. MAIBACH**. Transepidermal water loss (TEWL): Environment and pollution-A systematic review. *Skin Health Dis*, 2022, vol. 2 (2), e104
- Transepidermal Water Loss in Young and Aged Healthy Humans,. **KOTTNER, J ; A. VOGT ; A. LICHTERFELD ; U. BLUME-PEYTAVI**. Textbook of Aging Skin. Springer Berlin, 2015